# DEMANDE DE BREVET EUROPEEN

(11) **EP 1 655 019 A1**
(43) Date de publication de la demande: **10.05.2006**
(21) Numéro de dépôt: 05291060.1
(22) Date de dépôt: 17.05.2005
(51) Int. Cl.: A61K 8/44, A61K 8/87, A61K 8/891

(54) **Poudre cosmétique comprenant un organopolysiloxane élastomère**

(30) Priorité: 13.10.2004 US 617682
(71) Demandeur: L'OREAL, 75008 Paris (FR)
(72) Inventeur: Liechty Anne, 75013 Paris (FR); Thibout Camille, 94300 Vincennes (FR)
(74) Mandataire: Boulard, Denis

(57) **Abrégé**

L'invention a pour objet une poudre cosmétique comprenant une poudre d'organopolysiloxane réticulé élastomère, une charge sphérique additionnelle, notamment de polyuréthane ou de polyméthacrylate de méthyle, et éventuellement une poudre d'acide aminé N-acylé.

Application au maquillage et au soin de la peau.

## Description

La présente invention a pour objet une composition cosmétique de maquillage ou de soin de la peau sous forme de poudre comprenant une poudre d'organopolysiloxane élastomère associée à d'autres poudres. L'invention a également pour objet un procédé de maquillage ou de soin de la peau d'être humain comprenant l'application de la composition sur la peau.

La composition de maquillage selon l'invention est en particulier une composition de maquillage de la peau, telle qu'un fond de teint, un fard à paupières, un fard à joue, un produit anticernes, une poudre du visage et du corps, un produit de maquillage du corps. Plus spécialement, l'invention porte sur une composition de fond de teint.

La composition de soin de la peau peut être un produit de soin du visage, un produit de soin du corps, une poudre déodorante.

Les compositions de maquillage de la peau sont couramment employées pour apporter une couleur esthétique à la peau, notamment au visage, mais également pour camoufler les imperfections de la peau telles que les rougeurs, les taches, les rides.

Certaines compositions de maquillage se présentent sous la forme de poudre libre ou de poudre compactée. Ces compositions comprennent généralement une teneur en poudres élevée, notamment au moins 80 % en poids, par rapport au poids total de la composition. Selon le type de poudres utilisées, les propriétés cosmétiques du produit de maquillage peuvent être très variables. Or certaines poudres comme le carbonate de calcium, le carbonate de magnésium, le stéarate de magnésium ou le dioxyde de titane confèrent un toucher sec, rêche et nuisent à l'obtention de propriétés de douceur lors de la prise au doigt ou de l'étalement de la poudre cosmétique sur la peau. De plus, avec ces matières pulvérulentes, le dépôt de la poudre cosmétique sur la peau est opaque et couvrant et ne permet donc pas d'obtenir un maquillage transparent et camouflant ou estompant les imperfections du relief de la peau comme les microreliefs, les rides, les ridules. La poudre déposée sur la peau est très visible et présente un aspect poudreux prononcé qui n'est pas proche du grain naturel de la peau : le maquillage n'est pas naturel.

Le but de la présente invention est donc de disposer d'une composition de maquillage ou de soin de la peau sous forme de poudre ayant de bonnes propriétés de douceur lors de l'application sur la peau et permettant d'obtenir un dépôt, notamment un maquillage, transparent, camouflant les imperfections du relief de la peau comme les microreliefs, les rides, les ridules, tout en étant peu couvrant.

L'invention a également pour but de disposer d'une composition cosmétique sous forme de poudre permettant d'obtenir un dépôt sur la peau naturel et peu poudré laissant apparaître le grain naturel de la peau.

Les inventeurs ont découvert qu'une telle composition est obtenue en associant une poudre d'organopolysiloxane élastomère avec d'autres poudres particulières.

De façon plus précise, l'invention a pour objet une composition cosmétique sous forme de poudre comprenant une poudre d'organopolysiloxane réticulé élastomère, au moins une charge sphérique, et au moins une poudre d'acide aminé N-acylé.

L'invention a également pour objet une composition cosmétique sous forme de poudre comprenant une poudre d'organopolysiloxane réticulé élastomère, au moins une charge sphérique, la composition étant exempte de composés choisis parmi le carbonate de calcium, le carbonate de magnésium, l'hydro-carbonate de magnésium, les craies, le dioxyde de titane, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

L'invention a aussi pour objet une composition cosmétique sous forme de poudre comprenant au moins deux poudres d'organopolysiloxane élastomère et au moins une poudre d'acide aminé N-acylé.

L'invention a également pour objet une composition cosmétique sous forme de poudre comprenant une poudre d'organopolysiloxane élastomère, au moins une poudre de polyméthacrylate de méthyle, et au moins une poudre d'acide aminé N-acylé.

L'invention a encore pour objet une composition cosmétique sous forme de poudre comprenant une poudre d'organopolysiloxane élastomère et au moins une poudre de polyuréthane.

L'invention a également pour objet une composition cosmétique sous forme de poudre comprenant une poudre d'organopolysiloxane élastomère et au moins une poudre de polymère acrylique différente d'une poudre de polyméthacrylate de méthyle.

L'invention a également pour objet un procédé cosmétique de maquillage ou de traitement non thérapeutique de la peau comprenant l'application sur la peau d'une composition telle que définie précédemment.

L'invention a aussi pour objet l'utilisation d'une composition telle que définie précédemment pour obtenir un dépôt, notamment un maquillage, sur la peau transparent et/ou camouflant les imperfections du relief de la peau et/ou naturel et/ou laissant apparaître le grain de la peau.

La composition selon l'invention comprend une poudre d'organopolysiloxane élastomère, de préférence sphérique.

L' organopolysiloxane élastomère est avantageusement réticulé et peut être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A2) de diorganopolysiloxane contenant au moins deux hydrogènes liés chacun à un silicium, et (B2) de diorganopolysiloxane ayant au moins deux groupements à insaturation éthylénique liés au silicium, notamment en présence (C2) de catalyseur platine, comme par exemple décrit dans la demande EP-A-295886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A2) est le réactif de base pour la formation d'organopolysiloxane élastomère et la réticulation s'effectue par réaction d'addition du composé (A2) avec le composé (B2) en présence du catalyseur (C2).

Le composé (A2) est avantageusement un diorganopolysiloxane ayant au moins deux groupes alkényles inférieur (par exemple en C2-C4) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémités de la molécule organopolysiloxane. L'organopolysiloxane (A2) peut avoir une structure chaîne ramifiée, chaîne linéaire, cyclique ou réseau mais la structure chaîne linéaire est préférée. Le composé (A2) peut avoir une viscosité allant de l'état liquide à l'état de gomme. De préférence, le composé (A2) a une viscosité d'au moins 100 centistokes à 25 °C.
Les organopolysiloxanes (A2) peuvent être choisi parmi les méthylvinylsiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

Le composé (B2) est en particulier un organopolysiloxane ayant au moins 2 hydrogènes liés au silicium dans chaque molécule et est donc le réticulant du composé (A2).
Avantageusement, la somme du nombre de groupements éthyléniques par molécule du composé (A2) et le nombre d'atomes d'hydrogène liés au silicum par molécule du composé (B2) est d'au moins 4. Le composé (B2) peut être sous toute structure moléculaire, notamment de structure chaîne linéaire, chaîne ramifiée, structure cyclique.
Le composé (B2) peut avoir une viscosité à 25 °C allant de 1 à 50 000 centistokes, notamment pour être bien miscible avec le composé (A).
Il est avantageux que le composé (B2) soit ajouté en une quantité telle que le rapport moléculaire entre la quantité totale d'atomes d'hydrogène liés au silicium dans le composé (B2) et la quantité totale de tous les goupements à insaturation éthylénique dans le composé (A2) aille dans la gamme de 1/1 à 20/1.
Le composé (B2) peut être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (C2) est le catalyseur de la réaction de réticulation , et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C2) est de préférence ajouté de 0,1 à 1000 parts en poids, mieux de 1 à 100 parts en poids, en tant que métal platine propre pour 1000 parts en poids de la quantité totale des composés (A2) et (B2).

D'autres groupes organiques peuvent être liés au silicium dans les organopolysiloxane (A2) et (B2) décrits précédemment, comme par exemple des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitutés tel que phényléthyl ; et des groupes hydrocarbonés monovalents substitués tels que un groupe époxy, un gropue ester carboxylate, un groupe mercapto.

Avantageusement, l'organopolysiloxane élastomère est non émulsionnant. Le terme "non émulsionnant" définit des élastomères organopolysiloxane ne contenant pas de chaîne hydrophile telle que motifs polyoxyalkylènes ou polyglycérolés.

Des organopolysiloxanes élastomères sphériques sont notamment décrits dans les demandes JP-A-61-194009, EP-A-242219, EP-A-295886, EP-A-765656, dont le contenu est incorporé à titre de référence.

Comme poudres d'organopolysiloxanes élastomères, on peut utiliser celles vendues sous les dénominations "Dow Corning 9505 Powder", "Dow Corning 9506 Powder" par la société Dow Corning ; ces poudres ont pour nom INCI : dimethicone/vinyl dimethicone crosspolymer.

Avantageusement, la poudre d'organopolysiloxane élastomère peut comprendre au moins une poudre d'organopolysiloxane élastomère enrobée de résine de silicone, notamment de résine silsesquioxane, comme décrit par exemple dans le brevet US5538793 dont le contenu est incorporé à titre de référence. De telles poudre d'élastomères sont vendues sous les dénomination "KSP-100", "KSP-101", "KSP-102", "KSP-103", KSP-104", "KSP-105" par la société Shin Etsu, et ont pour nom lNCl : vinyl dimethicone/methicone silsesquioxane Crosspolymer.

D'autres organopolysiloxanes élastomères sous forme de poudres sphériques peuvent être des poudres de silicone hybride fonctionnalisé par des groupes fluoroalkyle, notamment vendues sous la dénomination "KSP-200" par la société Shin Etsu ; des poudres de silicones hybride fonctionnalisé par des groupes phényl, notamment vendues sous la dénomination "KSP-300" par la société Shin Etsu.

Ainsi, la composition peut comprendre avantageusement au moins deux poudres d'organopolysiloxane élastomère contenant au moins une poudre d'organopolysiloxane élastomère enrobée de résine de silicone, notamment de résine silsesquioxane, telle que décrite précédemment.

La poudre d'organopolysiloxane élastomère, en particulier non émulsionnant, de préférence sphérique, peut être présente dans la composition en une teneur allant de 5 % à 25 % en poids, par rapport au poids total de la composition, de préférence allant de 7 % à 15 % en poids, et préférentiellement allant de 8 % à 12 % en poids.

En particulier, la composition selon l'invention peut comprendre une poudre d'organopolysiloxane élastomère enrobée de résine de silicone, notamment de résine silsesquioxane, en une teneur allant de 1 % à 25 % en poids, par rapport au poids total de la composition, notamment de 1 % à 15 % en poids, de préférence allant de 2 % à 8 % en poids, et préférentiellement allant de 3 % à 7 % en poids.

La composition peut comprendre un mélange de poudre d'organopolysiloxane élastomère enrobée de résine de silicone, notamment de résine silsesquioxane, et de poudre d'organopolysiloxane élastomère non enrobé. Dans un tel mélange, la poudre d'organopolysiloxane élastomère enrobée de résine de silicone, notamment de résine silsesquioxane, peut être présente en une teneur allant de 1 % à 10 % en poids, par rapport au poids total de la composition, de préférence allant de 2 % à 8 % en poids, et préférentiellement allant de 3 % à 7 % en poids ; la poudre d'organopolysiloxane élastomère non enrobé peut être présente en une teneur allant de 1 % à 10 % en poids, par rapport au poids total de la composition, de préférence allant de 2 % à 8 % en poids, et préférentiellement allant de 3 % à 7 % en poids.

La composition selon l'invention peut comprendre avantageusement des charges sphériques. Ces charges sphériques peuvent être choisies parmi les charges minérales ou organiques, et de préférence parmi les charges organiques. Ces charges sphériques sont différentes des poudres d'organopolysiloxane élastomères décrites précédemment. Ces charges sont en particulier non élastomère. Avantageusement, ces charges ne sont pas filmogènes, c'est-à-dire qu'elles ne forment pas un film continu lorsqu'elles sont déposées sur un support tel que la peau.

Les charges sphériques peuvent être par exemples choisies parmi:
les poudres acryliques, notamment les poudres de polyméthacrylate de méthyle,
les poudres acryliques différentes des poudres de polyméthacrylate de méthyle ;
les poudres de polyuréthane,
ces poudres étant décrites ci-après.

La composition selon l'invention peut comprendre des charges sphériques en une teneur allant de 10 % à 40 % en poids, par rapport au poids total de la composition, de préférence allant de 15 % à 35 % en poids, préférentiellement allant de 15 % à 30 % en poids, plus préférentiellement allant de 15 % à 25 % en poids.

Selon un mode de réalisation de l'invention, la composition peut comprendre une poudre, en particulier sphérique, de polyméthacrylate de méthyle.

Les poudres de polyméthacrylate de méthyle se présentent généralement sous la forme de particules sphériques creuses ou pleines de couleur blanche dont la taille moyenne en nombre est généralement à l'échelle du micromètre, en particulier varie de 3 à 15 microns et généralement varie de 3 à 10 microns. Par « taille moyenne en nombre », on désigne la dimension donnée par la distribution granulométrique statistique à la moitié de la population, dite D50.

Il est également possible de caractériser ces particules de polyméthacrylate de méthyle par leur densité, celle-ci étant susceptible de varier notamment en fonction de la taille de la cavité sphérique desdites particules.
Dans le cadre de la présente invention, cette densité est appréciée selon le protocole suivant, dit de la densité tassée :
On verse m=40 g de poudre dans une éprouvette graduée ; puis on place l'éprouvette sur l'appareil STAV 2003 de chez STAMPF VOLUMETER ; l'éprouvette est ensuite soumise à 1500 tassements ; puis on mesure directement sur l'éprouvette le volume final Vf de poudre tassée. La densité tassée est déterminée par le rapport m/Vf, en l'occurrence 40/Vf (Vf étant exprimé en cm³ et m en g).
En particulier, la densité des particules de polyméthacrylate de méthyle utilisables selon l'invention peut varier de 0,3 à 1,5 , notamment de 0,5 à 1,5 , et plus particulièrement de 1 à 1,5.

A titre représentatif et non limitatif de polyméthacrylate de méthyle convenant à l'invention, on peut notamment citer les particules de polyméthacrylate de méthyle commercialisées par la société MATSUMOTO YUSHI CO sous la dénomination « Micropearl M100 », par la société LCW sous la dénomination « Covabead LH 85 » et celles commercialisées par la société NIHON JUNYAKU sous la dénomination « JURYMER MB1 ».

Les particules de polyméthacrylate de méthyle peuvent être présentes en une teneur allant de 5 % à 20 % en poids, par rapport au poids total de la composition, de préférence allant de 7 à 18 % en poids, et préférentiellement allant de 8 % à 15 % en poids.

La composition selon l'invention peut comprendre une poudre, en particulier sphérique, de polymère acrylique différente d'une poudre de polyméthacrylate de méthyle.

En particulier, la poudre acrylique peut être une poudre de polymère ou copolymère d'acrylonitrile , et en particulier des particules creuses expansées de polymère ou copolymère d'acrylonitrile. Avantageusement, les particules peuvent être réalisées en tout polymère ou copolymère d'acrylonitrile, expansé, non toxique et non irritant pour la peau.

En particulier, la masse volumique des particules est choisie dans la gamme allant de 15 kg/m³ à 200 kg/m³ et mieux de 40 kg/m³ à 120 kg/m³, et encore mieux de 60 kg/m³ à 80 kg/m³. Pour obtenir cette faible masse volumique, on utilise avantageusement des particules de polymères ou copolymères expansés de préférence à base d'acrylonitrile et d'un monomère acrylique ou styrénique et/ou de chlorure de vinylidène.

On peut par exemple utiliser un copolymère contenant : de 0 % à 60 % de motifs dérivés du chlorure de vinylidène, de 20 % à 90 % de motifs dérivés d'acrylonitrile et de 0 % à 50 % de motifs dérivés d'un monomère acrylique ou styrénique, la somme des pourcentages (en poids) étant égale à 100. Le monomère acrylique est par exemple un acrylate ou méthacrylate de méthyle ou d'éthyle. Le monomère styrénique est par exemple l'α-méthyl-styrène ou le styrène.

De préférence, les particules utilisées dans la présente invention sont des particules creuses d'un copolymère expansé de chlorure de vinylidène et d'acrylonitrile ou de chlorure de vinylidène, d'acrylonitrile et de méthacrylate. Ces particules peuvent être sèches ou hydratées.

Les particules de l'invention peuvent être obtenues, par exemple, selon les procédés des brevets et demandes de brevet EP-56219, EP-348372, EP-486080, EP-320473, EP-112807 et US-3615972.

La cavité interne des particules contient en principe un gaz qui peut être de l'air, de l'azote ou un hydrocarbure comme l'isobutane ou l'isopentane.

De façon avantageuse, les particules de l'invention ont une granulométrie allant de 1 µm à 80 µm et encore mieux allant de 10 µm à 50 µm, et encore mieux de 10 µm à 30 µm.

Les particules utilisables dans l'invention sont par exemple les microsphères de terpolymère expansé de chlorure de vinylidène, d'acrylonitrile et de méthacrylate, vendues sous la marque EXPANCEL par la société Expancel sous les références 551 DE 50 (granulométrie d'environ 40 µm), 551 DE 20 (granulométrie d'environ 30 µm et masse volumique d'environ 65 kg/m³), 551 DE 12 (granulométrie d'environ 12 µm), 551 DE 80 (granulométrie d'environ 80 µm), 461 DE 50 (granulométrie d'environ 50 µm). On peut aussi utiliser des microsphères formées du même terpolymère expansé ayant une granulométrie d'environ 18 µm et une masse volumique d'environ 70 kg/m³, appelées ci-dessous EL 23, ou ayant une granulométrie d'environ 34 µm et une masse volumique d'environ 20 kg/m³, appelées ci-dessous EL 43.

Avantageusement, ladite poudre acrylique est présente dans la composition lorsque celle-ci se présente sous la forme d'une poudre libre.

La poudre acrylique différente de la poudre de polyméthacrylate de méthyle peut être présente dans la composition selon l'invention en une teneur allant de 0,05 % à 2 % en poids, par rapport au poids total de la composition, de préférence allant de 0,1 % à 1,5 % en poids, et préférentiellement allant de 0,1 % à 1,2 % en poids.

La composition selon l'invention peut comprendre une poudre, en particulier sphérique, de polyuréthane. En particulier, la poudre de polyuréthane n'est pas filmogène, c'est-à-dire qu'elle ne forme pas un film continu lorsqu'elle est déposée sur un support tel que la peau.

Avantageusement, la poudre de polyuréthane est une poudre de copolymère de diisocyanate d'hexaméthylène et de triméthylol hexyl lactone. Une telle poudre de polyuréthane est notamment vendue sous les dénominations « PLASTIC POWDER D-400 », « PLASTIC POWDER D-800 » par la société TOSHIKI.

Comme autre poudre de polyuréthane, on peut utiliser celle vendue sous la dénomination « PLASTIC POWDER CS-400 » par la société TOSHIKI.

La poudre de polyuréthane peut être présente dans la composition selon l'invention en une teneur allant de 0,5 % à 30 % en poids, par rapport au poids total de la composition, et de préférence allant de 1 % à 15 % en poids, et préférentiellement allant de 5 % à 15 % en poids.

La composition selon l'invention peut comprendre une poudre d'acide aminé N-acylé. Une telle poudre confère une propriété crémeuse à la poudre cosmétique.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. L'acide aminé peut être par exemple la lysine, l'acide glutamique, l'alanine.

Avantageusement, la poudre d'acide aminé N-acylé peut être une poudre de lauroyl lysine.

La poudre d'acide aminé N-acylé peut être présente dans la composition selon l'invention en une teneur allant de 5 % à 20 % en poids, par rapport au poids total de la composition, de préférence allant de 7 à 18 % en poids, et préférentiellement allant de 8 % à 15 % en poids.

La composition selon l'invention peut comprendre du sulfate de baryum. Les particules de sulfate de baryum peuvent être enrobées d'acide aminé N-acylé tels que ceux décrits précédemment, et notamment enrobé de lauroyl lysine.

Le sulfate de baryum peut être présent dans la composition selon l'invention en une teneur allant de 1 % à 10 % en poids, par rapport au poids total de la composition, de préférence allant de 2 à 8 % en poids, et préférentiellement allant de 3 % à 7 % en poids.

La composition peut comprendre au moins une matière colorante pulvérulente pouvant être choisie parmi les pigments et les nacres.

Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

On peut également utiliser des pigments goniochromatiques ; ces pigments présentent un changement de couleur relativement important avec l'angle d'observation.

Le pigment goniochromatique peut être choisi par exemple parmi les pigments à structures multicouche interférentielles et les pigments à cristaux liquides.

Dans le cas d'une structure multicouche, celle-ci peut comporter par exemple au moins deux couches, chaque couche, indépendamment ou non de la (ou les) autre(s) couche(s), étant réalisée par exemple à partir d'au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.
Les agents goniochromatiques à structures multicouches sont notamment ceux décrits dans les documents suivants : US-A-3 438 796, EP-A-227423, US-A-5 135 812, EP-A-170439, EP-A-341002, US-A-4 930 866, US-A- 5 641 719, EP-A-472371, EP-A-395410, EP-A-753545, EP-A-768343, EP-A-571836, EP-A-708154, EP-A-579091, US-A-5 411 586, US-A- 5 364 467, WO-A-97/39066, DE-A-4 225 031, WO 9517479 (BASF), DE-A-196 14 637. Ils se présentent sous forme de paillettes, de couleur métallisée.

Les structures multicouches utilisables dans l'invention sont par exemple les structures suivantes : Al/SiO₂/Al/SiO₂/Al ; Cr/MgF₂/Al/MgF₂/Al ; MoS₂/SiO₂/Al/SiO₂/MoS₂ ; Fe₂O₃/SiO₂/Al/ SiO₂/Fe₂O₃ ; Fe₂O₃/SiO₂/Fe₂O₃/SiO₂/Fe₂O₃ ; MoS₂/SiO₂/mica-oxyde/SiO₂/MoS₂ ; Fe₂O₃/SiO₂/ mica-oxyde/SiO₂/Fe₂O₃. Selon l'épaisseur des différentes couches, on obtient différentes couleurs. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.
Par suite la structure multicouche peut être essentiellement minérale ou organique. Selon l'épaisseur de chacune des différentes couches, on obtient différentes couleurs.

Les pigments goniochromatiques à structure multicouche interférentielle selon l'invention sont notamment ceux décrits dans les documents suivants : US-A-3 438 796, EP-A-227423, US-A-5 135 812, EP-A-170439, EP-A-341002, US-A-4 930 866, US-A- 5 641 719, EP-A-472371, EP-A-395410, EP-A-753545, EP-A-768343, EP-A-571836, EP-A-708154, EP-A-579091, US-A-5 411 586, US-A- 5 364 467, WO-A-97/39066, DE-A-4 225 031, WO 9517479 (BASF), DE-A-196 14 637, et leurs associations. Ils se présentent sous forme de paillettes, de couleur métallisée.

De préférence, le pigment goniochromatique à structure multicouche interférentielle peut être choisi dans le groupe constitué par les pigments goniochromatiques commerciaux suivants : Infinite Colors de la société Sihiseido, Sicopearl Fantastico de BASF, Colorstream, Xirallic, Xirona de MERCK, Colorglitter de FLEX, et leurs mélanges.

Comme pigment goniochromatique à structure multicouche, on peut citer ceux vendus sous la dénomination "SICOPEARL

Des pigments à cristaux liquides sont notamment décrits dans la demande EP-A-1046692.

Comme particules à cristaux liquides, on peut notamment utiliser celles connues sous le nom CTFA Polyacrylate-4 et vendus sous les dénominations «HELICONE® HC Sapphire », «HELICONE® HC Scarabeus », «HELICONE® HC Jade », «HELICONE® HC Maple », «HELICONE® HC XL Sapphire », « HELICONE® HC XL Scarabeus », « HELICONE® HC XL Jade », « HELICONE® HC XL Maple » par la société WACKER.

Les matières colorantes peuvent être présentes dans la composition selon l'invention en une teneur allant de 0 ,5 % à 40 % en poids, par rapport au poids total de la composition, de préférence allant de 1 % à 30 % en poids, et préférentiellement allant de 3 % à 25 % en poids.

La composition selon l'invention peut comprendre au moins une ou des charge(s) additionnelle(s), différente(s) des poudres d'organopolysiloxane élastomère et des charges sphériques décrites précédemment.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique ( par exemple feuillet, cubique, hexagonale, orthorombique, etc) . On peut citer le talc, le mica, la silice, le kaolin, les poudres de polyamide (Nylon®), les poudres de poly-β-alanine, les poudres de polyéthylène, les poudres de polymères de tétrafluoroéthylène (Téflon®), l'amidon, le nitrure de bore, les poudres de résine de silicone (Tospearls® de Toshiba, par exemple), l'hydroxyapatite, la séricite, les billes de verre, les billes de céramique.

La charge additionnelle peuvent être présente dans la composition en une teneur allant de 0,5 % à 75 % en poids, par rapport au poids total de la composition, de préférence allant de 1 % à 60 % en poids, et préférentiellement allant de 5 % à 60 % en poids.

Avantageusement, la composition selon l'invention est exempte de composés choisis parmi le carbonate de calcium, le carbonate de magnésium, l'hydrocarbonate de magnésium, les craies, le dioxyde de titane, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium. De tels composés ont l'inconvénient de nuire au toucher doux de la poudre cosmétique.
Par « exempte », on entend que la composition contient moins de 4 % en poids, par rapport au poids total de la composition, desdits composés exempts, de préférence moins de 3 % en poids, de préférence moins de 2 % en poids, préférentiellement moins de 1 % en poids, voire ne contient pas ( soit 0 % en poids) de tels composés.

La composition selon l'invention peut comprendre avantageusement une teneur totale en composés pulvérulents allant de 80 % à 99 % en poids, par rapport au poids total de la composition, et de préférence allant de 85 % à 99 % en poids.

La composition selon l'invention peut comprendre avantageusement au moins une phase grasse qui généralement comprend au moins une huile. Ce type de phase grasse est encore communément appelé liant et sert notamment de milieu dispersant pour la phase particulaire.

L'huile peut être choisie parmi les huiles utilisées classiquement comme liant dans les poudres libres ou compactes. Ces huiles peuvent être notamment choisies parmi :
- l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ;
- les huiles d'hydrocarbures, telles que les huiles de paraffine, le squalane, la vaseline ;
- les esters gras, tels que le myristate d'isopropyle, le palmitate d'isopropyle, le stéarate de butyle, le stéarate d'isodécyle, le stéarate d'isocétyle, le laurate d'hexyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ;
- les huiles de silicone telles que les polyméthylsiloxanes, les polyméthylphénylsiloxanes, les polysiloxanes modifiés par des acides gras, des alcools gras ou des polyoxyalkylènes, les silicones fluorées ou les huiles perfluorées ;
- les acides gras supérieurs tels que l'acide myristique, l'acide palmitique, l'acide stéarique, l'acide béhénique, l'acide oléique, l'acide linoléique, l'acide linolénique ou l'acide isostéarique;
- les alcools gras supérieurs tels que le cétanol, l'alcool stéarylique ou l'alcool oléique ;
- les poly méthylfluoroalkyl diméthylsiloxanes de formule (I) : 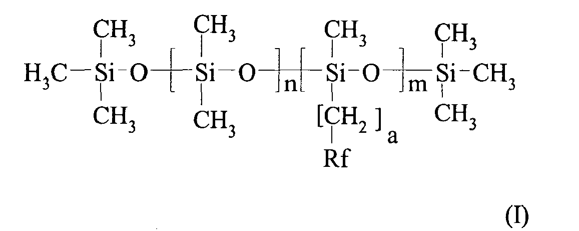
dans laquelle :
- n représente un entier variant de 5 à 90, notamment de 30 à 80 et en particulier de 50 à 80,
- m représente un entier variant de 1 à 150, notamment de 1 à 80, et en particulier de 1 à 40,
- a représente un entier variant de 0 à 5, et
- Rf désigne un radical perfluoroalkyle comprenant de 1 à 8 atomes de carbone ; et
- leurs mélanges.

Comme composés de formule (I) convenant tout particulièrement à l'invention, on peut notamment citer ceux vendus sous la dénomination X22-819, X22-820, X22-821, X22-822 par la société SHIN-ETSU.

La composition selon l'invention peut comprendre au moins une huile en une teneur allant de 1 à 20 % en poids, et notamment de 2 à 15 % en poids par rapport au poids total de la composition.

La composition peut contenir d'autres ingrédients cosmétiques usuels pouvant être choisis notamment parmi les antioxydants, les parfums, les conservateurs, les neutralisants, les tensioactifs, les cires, l'eau, les filtres solaires, les vitamines, les hydratants, les composés auto-bronzants, les actifs antirides.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Avantageusement, la composition selon l'invention est une composition anhydre. On entend par composition anhydre une composition contenant moins de 2 % en poids d'eau, voire moins de 0,5 % d'eau, et notamment exempte d'eau, l'eau n'étant pas ajoutée lors de la préparation de la composition mais correspondant à l'eau résiduelle apportée par les ingrédients mélangés.

La composition selon l'invention peut se présenter sous la forme d'une poudre libre ou d'une poudre compacte. Une poudre compacte désigne une poudre pressée à l'aide d'une presse manuelle ou mécanique.

L'invention est illustrée plus en détails par les exemples décrits ci-après.

### Exemple 1 :

On a préparé une poudre libre pour le visage ayant la composition suivante :

| | |
|---|---|
| Particules de polydiméthylsiloxane réticulé enrobé de résine de silicone (KSP-100 de la société Shin Etsu) | 5 g |
| Poudre sphérique de silicone élastomère (Dow Corning 9506 Powder de Dow Corning) | 5 g |
| Poudre sphérique de polyuréthane (Plastic Powder D-400 de Toshiki) | 10 g |
| Poudre sphérique de polyméthacrylate de méthyle (Ganzpearl GMP 0820 de Ganz Chemical) | 10 g |
| Micrsophères de copolymère de chlorure de vinylidène/acrylonitrile/ méthacrylate de méthyle expansées à l'isobutane (Expancel 551 DE 20 D60 de la société Expancel) | 0,50 g |
| Lauroyl lysine | 5 g |
| Sulfate de baryum enrobé lauroyl lysine ( LLD-5 BaSO4 de Daito Kasei) | 5 g |
| Talc | 37,6 g |
| Mica | 15 g |
| Oxydes de fer | 3,30 g |
| Conservateurs | 0,6 g |
| Stéarate d'isocétyle | 3 g |

La poudre s'étale facilement sur le visage et présente de bonnes propriétés de douceur. Le maquillage obtenu est transparent, homogène, unifiant, présente un aspect naturel laissant apparaître le grain de la peau et estompe les défauts de relief de la peau.

### Exemple 2 :

On a préparé une poudre libre pour le visage ayant la composition suivante :

| | |
|---|---|
| Particules de polydiméthylsiloxane réticulé enrobé de résine de silicone (KSP-100 de la société Shin Etsu) | 5 g |
| Poudre sphérique de silicone élastomère (Dow Corning 9506 Powder de Dow Corning) | 5 g |
| Poudre sphérique de polyuréthane (Plastic Powder D-400 de Toshiki) | 10 g |
| Poudre sphérique de polyméthacrylate de méthyle (Ganzpearl GMP 0820 de Ganz Chemical) | 10 g |
| Micrsophères de copolymère de chlorure de vinylidène/acrylonitrile/ méthacrylate de méthyle expansées à l'isobutane (Expancel 551 DE 20 D60 de la société Expancel) | 0,50 g |
| Lauroyl lysine | 5 g |
| Sulfate de baryum enrobé lauroyl lysine ( LLD-5 BaS04 de Daito Kasei) | 5 g |
| Talc | 12,7 g |
| Mica | 12 g |
| Oxydes de fer | 5,2 g |
| Pigments nacrés | 26 g |
| Conservateurs | 0,6 g |
| Stéarate d'isocétyle | 3 g |

La poudre s'étale facilement sur le visage et présente de bonnes propriétés de douceur. Le maquillage obtenu est transparent, homogène, unifiant, présente un aspect naturel laissant apparaître le grain de la peau et estompe les défauts de relief de la peau.

### Exemple 3 :

On a préparé une poudre libre pour le visage ayant la composition suivante :

| | |
|---|---|
| Particules de polydiméthylsiloxane réticulé enrobé de résine de silicone (KSP-100 de la société Shin Etsu) | 20 g |
| Poudre sphérique de polyuréthane (Plastic Powder D-400 de Toshiki ) | 10 g |
| Poudre sphérique de polyméthacrylate de méthyle (Ganzpearl GMP 0820 de Ganz Chemical) | 10 g |
| Micrsophères de copolymère de chlorure de vinylidène/acrylonitrile/ méthacrylate de méthyle expansées à l'isobutane (Expancel 551 DE 20 D60 de la société Expancel) | 0,50 g |
| Lauroyl lysine | 5 g |
| Sulfate de baryum enrobé lauroyl lysine ( LLD-5 BaSO4 de Daito Kasei) | 5 g |
| Talc | 27,6 g |
| Mica | 15 g |
| Oxydes de fer | 3,30 g |
| Conservateurs | 0,6 g |
| Stéarate d'isocétyle | 3 g |

La poudre s'étale facilement sur le visage et présente de bonnes propriétés de douceur. Le maquillage obtenu est transparent, homogène, unifiant, présente un aspect naturel laissant apparaître le grain de la peau et estompe les défauts de relief de la peau.

### Exemple 4 :

On a préparé une poudre compacte pour le visage ayant la composition suivante :

| | |
|---|---|
| Particules de polydiméthylsiloxane réticulé enrobé de résine de silicone (KSP-100 de la société Shin Etsu) | 5 g |
| Poudre sphérique de silicone élastomère (Dow Corning 9506 Powder de Dow Corning) | 5 g |
| Poudre sphérique de polyuréthane (Plastic Powder D-400 de Toshiki ) | 10 g |
| Poudre sphérique de polyméthacrylate de méthyle (Ganzpearl GMP 0820 de Ganz Chemical) | 10 g |
| Micrsophères de copolymère de chlorure de vinylidène/acrylonitrile/ méthacrylate de méthyle expansées à l'isobutane (Expancel 551 DE 20 D60 de la société Expancel) | 0,50 g |
| Lauroyl lysine | 5 g |
| Sulfate de baryum enrobé lauroyl lysine ( LLD-5 BaS04 de Daito Kasei) | 5 g |
| Talc | 21,2 g |
| Mica | 10 g |
| Oxydes de fer | 3,30 g |
| Stéarate de zinc | 3 g |
| Oxychlorure de bismuth | 10 g |
| Conservateurs | 1,4 g |
| Phenyl trimethicone | 6 g |
| Triisostéarate de glycérol | 6 g |

La poudre est tamisée, versée dans une coupelle métallique puis compactée.
La poudre s'étale facilement sur le visage et présente de bonnes propriétés de douceur. Le maquillage obtenu est transparent, homogène, unifiant, présente un aspect naturel laissant apparaître le grain de la peau et estompe les défauts de relief de la peau.

## Revendications

1. Composition cosmétique sous forme de poudre comprenant une poudre d'organopolysiloxane élastomère, au moins une charge sphérique, et au moins une poudre d'acide aminé N-acylé.

2. Composition cosmétique sous forme de poudre comprenant une poudre d'organopolysiloxane élastomère, au moins une charge sphérique additionnelle, la composition étant exempte de composés choisis parmi le carbonate de calcium, le carbonate de magnésium, l'hydro-carbonate de magnésium, les craies, le dioxyde de titane, les savons métalliques dérivés d'acides organiques carboxyliques ayant de 8 à 22 atomes de carbone.

3. Composition cosmétique sous forme de poudre comprenant au moins deux poudres d'organopolysiloxane élastomère, et au moins une poudre d'acide aminé N-acylé.

4. Composition cosmétique sous forme de poudre comprenant une poudre d'organopolysiloxane élastomère, au moins une poudre de polyméthacrylate de méthyle, et au moins une poudre d'acide aminé N-acylé.

5. Composition cosmétique sous forme de poudre comprenant une poudre d'organopolysiloxane élastomère et au moins une poudre de polyuréthane.

6. Composition cosmétique sous forme de poudre comprenant une poudre d'organopolysiloxane élastomère et au moins une poudre de polymère acrylique différente d'une poudre de polyméthacrylate de méthyle.

7. Composition selon l'une des revendications précédentes, **caractérisée par le fait que** l'organopolysiloxane élastomère est choisi parmi ceux obtenus :
- par réaction d'addition réticulation de diorganosiloxane contenant au moins un hydrogène lié au silicium et de diorganopolysiloxane ayant des groupements à insaturation éthylénique liés au silicium ;
- par réaction de condensation réticulation déhydrogénation entre un diorganopolysiloxane à terminaisons hydroxyle et un diorganopolysiloxane contenant au moins un hydrogène lié au silicium ;
- par réaction de condensation réticulation d'un diorganopolysiloxane à terminaisons hydroxyle et d'un organopolysilane hydrolysable ;
- par réticulation thermique d'organopolysiloxane ;
- par réticulation d'organopolysiloxane par radiations de haute énergie.

8. Composition selon l'une quelconque des revendications précédentes, **caractérisée par le fait que** l'organopolysiloxane élastomère est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique liés au silicium, notamment en présence (C) de catalyseur platine.

9. Composition selon l'une quelconque des revendications précédentes, **caractérisée par le fait que** l'organopolysiloxane élastomère est obtenu par réaction de diméthylpolysiloxane à terminaisons diméthylvinylsiloxy et de méthylhydrogénopolysiloxane à terminaisons triméthylsiloxy, en présence de catalyseur platine.

10. Composition selon l'une quelconque des revendications précédentes, **caractérisée par le fait que** l'organopolysiloxane réticulé est un élastomère non émulsionnant.

11. Composition selon l'une quelconque des revendications précédentes, **caractérisée par le fait que** la poudre d'organopolysiloxane élastomère comprend une poudre d'organopolysiloxane élastomère enrobée de résine de silicone, notamment de résine silsesquioxane.

12. Composition selon l'une quelconque des revendications précédentes, **caractérisée par le fait que** la poudre d'organopolysiloxane élastomère est une poudre sphérique.

13. ; Composition selon l'une quelconque des revendications précédentes, **caractérisée par le fait que** la poudre d'organopolysiloxane est présente en une teneur allant de 5 % à 25 % en poids, par rapport au poids total de la composition, de préférence allant de 7 % à 15 % en poids, et préférentiellement allant de 8 % à 12 % en poids.

14. Composition selon la revendication 11, **caractérisée par le fait que** la poudre d'organopolysiloxane élastomère enrobée de résine de silicone, notamment de résine silsesquioxane, est présente en une teneur allant de 1 % à 25 % en poids, par rapport au poids total de la composition, de préférence allant de 2 % à 8 % en poids, et préférentiellement allant de 3 % à 7 % en poids.

15. Composition selon l'une quelconque des revendications précédentes, **caractérisée par le fait qu'**elle comprend un mélange de poudre d'organopolysiloxane élastomère enrobée de résine de silicone, notamment de résine silsesquioxane, et de poudre d'organopolysiloxane élastomère non enrobé.

16. Composition selon la revendication précédente, **caractérisée par le fait que** la poudre d'organopolysiloxane élastomère non enrobé est présente en une teneur allant de 1 % à 10 % en poids, par rapport au poids total de la composition, de préférence allant de 2 % à 8 % en poids, et préférentiellement allant de 3 % à 7 % en poids.

17. Composition selon la revendication 15, **caractérisée par le fait que** la poudre d'organopolysiloxane élastomère enrobée de résine de silicone est présente en une teneur allant de 1 % à 10 % en poids, par rapport au poids total de la composition, de préférence allant de 2 % à 8 % en poids, et préférentiellement allant de 3 % à 7 % en poids.

18. Composition selon la revendication 3, **caractérisée par le fait qu'**elle comprend au moins une charge sphérique.

19. Composition selon l'une quelconque des revendications 1, 2 et 18, **caractérisée par le fait que** les charges sphériques sont choisies parmi les charges organiques.

20. Composition selon l'une quelconque des revendications 1, 2, 18, 19, **caractérisée par le fait que** les charges sphériques sont choisies parmi les poudres de polyméthacrylate de méthyle, les poudres acryliques différentes des poudres de polyméthacrylate de méthyle, les poudres de polyuréthane.

21. Composition selon l'une quelconque des revendications 1, 2 et 18 à 20, **caractérisée par le fait que** les charges sphériques sont présentes en une teneur allant de 10 % à 40 % en poids, par rapport au poids total de la composition, de préférence allant de 15 % à 35 % en poids, préférentiellement allant de 15 % à 30 % en poids, plus préférentiellement allant de 15 % à 25 % en poids.

22. Composition selon l'une quelconque des revendications 1 à 3 et 5, 6, **caractérisée par le fait qu'**elle comprend une poudre de polyméthacrylate de méthyle.

23. Composition selon la revendication précédente, **caractérisée par le fait que** la poudre de polyméthacrylate de méthyle a une densité allant de 0,3 à 1,5 , notamment de 0,5 à 1,5 , et plus particulièrement de 1 à 1,5.

24. Composition selon l'une quelconque des revendications 1 à 3, 5, 6, 22, 23, **caractérisée par le fait que** la poudre de polyméthacrylate de méthyle est présente en une teneur allant de 5 % à 20 % en poids, par rapport au poids total de la composition, de préférence allant de 7 à 18 % en poids, et préférentiellement allant de 8 % à 15 % en poids.

25. Composition selon l'une quelconque des revendications 1 à 5, **caractérisée par** le fait comprend une poudre de polymère acrylique différente d'une poudre de polyméthacrylate de méthyle.

26. Composition selon l'une quelconque des revendications 6 et 25, **caractérisée par le fait que** la poudre de polymère acrylique est une poudre de polymère ou copolymère d'acrylonitrile.

27. Composition selon l'une quelconque des revendications 6, 25, 26, **caractérisée par le fait que** la poudre de polymère acrylique comprend des particules creuses expansées de polymère ou copolymère d'acrylonitrile.

28. Composition selon l'une quelconque des revendications 6, 25 à 27, **caractérisée par le fait que** la poudre de polymère acrylique comprend des particules ayant une masse volumique allant de 15 kg/m³ à 200 kg/m³ et mieux de 40 kg/m³ à 120 kg/m³, et de préférence allant de 60 kg/m³ à 80 kg/m³.

29. Composition selon l'une quelconque des revendications 6, 25 à 28, **caractérisée par le fait que** le polymère acrylique est un copolymère d'acrylonitrile et d'un monomère acrylique ou styrénique et/ou de chlorure de vinylidène.

30. Composition selon l'une quelconque des revendications 6, 25 à 29, **caractérisée par le fait que** le polymère acrylique est un copolymère contenant : de 0 % à 60 % de motifs dérivés du chlorure de vinylidène, de 20 % à 90 % de motifs dérivés d'acrylonitrile et de 0 % à 50 % de motifs dérivés d'un monomère acrylique ou styrénique, la somme des pourcentages (en poids) étant égale à 100.

31. Composition selon la revendication précédente, **caractérisée par le fait que** le monomère acrylique est choisi parmi le (méth)acrylate de méthyle et le (méth)acrylate d'éthyle.

32. Composition selon la revendication 29, **caractérisée par le fait que** le monomère styrénique est l'α-méthyl-styrène ou le styrène.

33. Composition selon l'une quelconque des revendications 6, 25 à 32, **caractérisée par le fait que** la poudre de polymère acrylique comprend des particules creuses d'un copolymère expansé de chlorure de vinylidène et d'acrylonitrile ou de chlorure de vinylidène, d'acrylonitrile et de méthacrylate.

34. Composition selon l'une quelconque des revendications 6, 25 à 33, **caractérisée par le fait que** la poudre de polymère acrylique comprend des particules ayant une cavité interne des particules contenant un gaz choisi parmi l'air, l'azote, l'isobutane, l'isopentane.

35. Composition selon l'une quelconque des revendications 6, 25 à 34, **caractérisée par le fait que** la poudre de polymère acrylique comprend des particules ayant une granulométrie allant de 1 µm à 80 µm, de préférence allant de 10 µm à 50 µm, et préférentiellement allant de 10 µm à 30 µm.

36. Composition selon l'une quelconque des revendications 6, 25 à 35, **caractérisée par le fait que** la poudre acrylique différente de la poudre de polyméthacrylate de méthyle est présente en une teneur allant de 0,05 % à 2 % en poids, par rapport au poids total de la composition, de préférence allant de 0,1 % à 1,5 % en poids, et préférentiellement allant de 0,1 % à 1,2 % en poids.

37. Composition selon l'une quelconque des revendications 1 à 4 et 6, **caractérisée par le fait qu'**elle comprend une poudre de polyuréthane.

38. Composition selon la revendications 1 à 4, 6, 37, **caractérisée par le fait que** la poudre de polyuréthane est une poudre de copolymère de diisocyanate d'hexaméthylène et de triméthylol hexyl lactone.

39. Composition selon la revendication 1 à 4, 6, 37, 38, **caractérisée par le fait que** la poudre de polyuréthane est présente en une teneur allant de 0,5 % à 30 % en poids, par rapport au poids total de la composition, et de préférence allant de 1 % à 15 % en poids, et préférentiellement allant de 5 % à 15 % en poids.

40. Composition selon l'une quelconque des revendications 2, 5, 6, **caractérisée par le fait qu'**elle comprend une poudre d'acide aminé N-acylé.

41. Composition selon l'une quelconque des revendications 1, 3, 4, 40, **caractérisée par le fait que** l'acide aminé N-acylé comprend un groupe acyle ayant de 8 à 22 atomes de carbones.

42. Composition selon l'une quelconque des revendications 40 ou 41, **caractérisée par le fait que** l'acide aminé N-acylé comprend un groupe acyle choisi parmi les gropues 2-éthyl hexanoyle, caproyle, lauroyle, myristoyle, palmitoyle, stéaroyle, cocoyle.

43. Composition selon la revendication précédente, **caractérisée par le fait que** l'acide aminé est choisi parmi la lysine, l'acide glutamique, l'alanine.

44. Composition selon l'une quelconque des revendications 41 à 43, **caractérisée par le fait que** l'acide aminé N-acylé est la lauroyl lysine.

45. Composition selon l'une quelconque des revendications 1, 3, 4, 40 à 44, **caractérisée par le fait que** la poudre d'acide aminé N-acylé est présente en une teneur allant de 5 % à 20 % en poids, par rapport au poids total de la composition, de préférence allant de 7 à 18 % en poids, et préférentiellement allant de 8 % à 15 % en poids.

46. Composition selon l'une quelconque des revendications précédentes, **caractérisée par le fait qu'**elle comprend du sulfate de baryum.

47. Composition selon la revendication précédente, **caractérisée par le fait que** le sulfate de baryum est enrobé d'acide aminé N-acylé.

48. Composition selon la revendication précédente, **caractérisée par le fait que** l'acide aminé N-acylé est conforme selon l'une quelconque des revendications 41 à 44.

49. Composition selon l'une quelconque des revendications 46 à 48, **caractérisée par le fait que** le sulfate de baryum est enrobé de lauroyl lysine.

50. Composition selon l'une quelconque des revendications 46 à 49, **caractérisée par le fait que** le sulfate de baryum est présent en une teneur allant de 1 % à 10 % en poids, par rapport au poids total de la composition, de préférence allant de 2 à 8 % en poids, et préférentiellement allant de 3 % à 7 % en poids.

51. Composition selon l'une quelconque des revendications précédentes, **caractérisée par le fait qu'**elle comprend au moins une matière colorante pulvérulente.

52. Composition selon la revendication précédente, **caractérisée par le fait que** la matière colorante pulvérulente est choisie parmi les pigments et les nacres.

53. Composition selon la revendication précédente, **caractérisée par le fait que** la matière colorante pulvérulente est présente en une teneur allant de 0 ,5 % à 40 % en poids, par rapport au poids total de la composition, de préférence allant de 1 % à 30 % en poids, et préférentiellement allant de 3 % à 25 % en poids.

54. Composition selon l'une quelconque des revendications précédentes, **caractérisée par le fait qu'**elle comprend au moins une charge additionnelle.

55. Composition selon la revendication précédente, **caractérisée par le fait que** la charge additionnelle est choisie parmi le talc, le mica, la silice, le kaolin, les poudres de polyamide, les poudres de poly-β-alanine, les poudres de polyéthylène, les poudres de polymères de tétrafluoroéthylène, l'amidon, le nitrure de bore, les poudres de résine de silicone, l'hydroxyapatite, la séricite, les billes de verre, les billes de céramique.

56. Composition selon l'une quelconque des revendications 54 ou 55, **caractérisée par le fait que** la charge additionnelle est présente en une teneur allant de 0,5 % à 75 % en poids, par rapport au poids total de la composition, de préférence allant de 1 % à 60 % en poids, et préférentiellement allant de 5 % à 60 % en poids.

57. Composition selon l'une quelconque des revendications 1 et 3 à 56, **caractérisée par le fait qu'**elle est exempte de composés choisis parmi le carbonate de calcium, le carbonate de magnésium, l'hydro-carbonate de magnésium, les craies, le dioxyde de titane, les savons métalliques dérivés d'acides organiques carboxyliques ayant de 8 à 22 atomes de carbone.

58. Composition selon l'une quelconque des revendications précédentes, **caractérisée par le fait qu'**elle comprend une teneur totale en composés pulvérulents allant de 80 % à 99 % en poids, par rapport au poids total de la composition, et de préférence allant de 85 % à 99 % en poids.

59. Composition selon l'une quelconque des revendications précédentes, **caractérisée par** le fait quelle comprend au moins une huile.

60. Composition selon la revendication précédente, **caractérisée par le fait que** l'huile est présente en une teneur allant de 1 à 20 % en poids, et notamment de 2 à 15 % en poids par rapport au poids total de la composition.

61. Composition selon l'une quelconque des revendications précédentes, **caractérisée par le fait qu'**elle comprend un ingrédient cosmétique choisi parmi les antioxydants, les parfums, les conservateurs, les neutralisants, les tensioactifs, les cires, l'eau, les filtres solaires, les vitamines, les hydratants, les composés auto-bronzants, les actifs antirides, les actifs déodorants.

62. Composition selon l'une quelconque des revendications précédentes, **caractérisée par le fait qu'**elle est anhydre.

63. Composition selon l'une quelconque des revendications précédentes, **caractérisée par le fait qu'**elle se présente sous la forme d'une poudre libre ou d'une poudre compacte.

64. Composition selon l'une quelconque des revendications précédentes, **caractérisée par le fait qu'**elle est une poudre de maquillage ou une poudre de soin de la peau.

65. Composition selon l'une quelconque des revendications précédentes, **caractérisée par le fait que** la composition est sous la forme d'un fond de teint, d'un fard à paupières, d'un fard à joue, d'un produit anticernes, d'un produit de maquillage du corps, d'un produit de soin du visage, d'un produit de soin du corps, d'une poudre déodorante.

66. Procédé cosmétique de maquillage ou de traitement thérapeutique de la peau comprenant l'application sur la peau d'une composition selon l'une quelconque des revendications précédentes.

67. Utilisation d'une composition selon l'une quelconque des revendications 1 à 65 pour obtenir un dépôt, notamment un maquillage, sur la peau transparent et/ou camouflant les imperfections du relief de la peau et/ou naturel et/ou laissant apparaître le grain de la peau.
